Europäisches Patentamt

European Patent Office

Office européen des brevets

(11) Publication number: **0 134 489**
**A1**

## (12) EUROPEAN PATENT APPLICATION

(21) Application number: **84108120.1**

(22) Date of filing: **11.07.84**

(51) Int. Cl.⁴: **A 61 F 2/16**

(30) Priority: **20.07.83 US 515521**

(43) Date of publication of application:
**20.03.85 Bulletin 85/12**

(84) Designated Contracting States:
**AT BE CH DE FR GB IT LI LU NL SE**

(71) Applicant: **McTigue, John**
**1757 P Street, N.W.**
**Washington, D.C. 20009(US)**

(72) Inventor: **McTigue, John**
**1757 P Street, N.W.**
**Washington, D.C. 20009(US)**

(74) Representative: **Goddar, Heinz J., Dr. et al,**
**FORRESTER & BOEHMERT Widenmayerstrasse 4/I**
**D-8000 München 22(DE)**

(54) **Intraocular lens.**

(57) An intraocular lens (10) which includes a double plano surface is shown and described. A first posterior surface (14) is used as a lens to focus light on a patient's retina, the second posterior surface (16) is used to provide a continuous sealing contact with the patient's posterior lens capsule (18) at all time.

A method of performing an intraocular lens implant by the use of a lens which seals the patients posterior lens capsule to the lens and which is suitable for discission by laser radiation or surgery after the implantation is completed is shown and described.

FIG. 4

Croydon Printing Company Ltd

EP 0 134 489 A1

INTRAOCULAR LENS

BACKGROUND OF THE INVENTION

1.  Field of the invention

This invention relates to an intraocular lens and more particularly a posterior chamber lens which is adapted to  maintain continuous sealing contact with the patient's posterior lens capsule.

2.  Description of the prior art

In the correction of a cataract condition a surgical technique known as extra capsular cataract extraction may be employed.  In this procedure, an incision is made through the patient's anterior capsule and . the patient's lens with clouded cellular material is removed.  Various scrapping, suction, or phacoemulsification techniques are used to accomplish such extraction.  The rear capsule wall (the "posterior capsule") remains in place in the eye.

When a replacement intraocular lens is installed, without discission of the patient's posterior capsule, the posterior capsule may lie against the intraocular lens.  As long as the posterior lens portion remains clear and unclouded, there is no need to perform a discission on the posterior portion.  However, when the posterior lens portion becomes clouded, it is necessary to perform a discission on the posterior portion.

Posterior discission in the prior art has been by means of laser cutting of the posterior capsule or by cutting of the posterior capsule by means of a knife or other instrument.

The procedure relying upon the laser requires that the laser beam be concentrated on the posterior capsule. Intraocular lens damage may result where the posterior capsule is in direct contact with the implanted lens at the point where it is desired to perform the discission. In such instances, the energy of the laser is concentrated in the very same region as the implanted lens surface, and this may cause pitting or other deterioration of the implanted lens.

In the technique utilizing a knife or other medical instrument, two procedures are known. In U.S. Patent 4,244,060 to Hoffer there is shown and described a technique wherein a portion of the implanted lens maintains a separation between the implanted lens and the patient's posterior capsule. Hoffer also describes the use of an opening in the portion of the lens used for maintaining separation. This allows for passage of the knife used for discission. This results in the undesirable condition where vitreous fluid may actually pass through the discission and through the opening used for maintaining separation which was required to perform the discission.

In lenses of the type described wherein the patient's posterior capsule lies immediately adjacent the implanted lens, discission may also be performed by a separate insertion of the discission knife into the region of the eye containing the vitreous fluid. A cut is made in the patient's posterior lens capsule behind the implanted lens. This procedure requires a further disturbance of the posterior portion of the eye and hence increases the risk of failure of the cataract correction operation.

The use of plano-convex lenses for intraocular lens implants is well known as is illustrated in U.S. Patents 2,444,060, 4,328,595, 4,110,848, 4,010,496, 4,340,979 and 4,251,887. The above referenced patents relate to the use plano-convex lenses with various devices to secure the lens after extra capsular cataract extraction.

## SUMMARY OF THE INVENTION

In this invention there is provided a means for maintaining a continuous complete seal around the periphery of the implanted lens with the patient's posterior capsule. In the preferred embodiment this is a second plano surface which lies behind or posterially of the first plano surface of the lens.

It is an object of this invention to maintain a separation between the patient's posterior capsule and the central region or optical portion of the lens. By maintenance of this separation discission of the patient's posterior capsule may be performed subsequent to lens implantation by means of a laser. The separation or holding the posterior lens capsule portion back and away from the plano optical surface of the lens allows for laser discission without danger of pitting or other undesirable effects on the lens.

Another object of this invention is to prevent flow of vitreous fluid from the posterior area of the eye to the anterior portion which lies forward of the implanted lens. This object is accomplished by maintaining a continuous seal between the lens and the patient's posterior lens capsule outer periphery at all times, even after discission of the central region of the lens capsule.

## BRIEF DESCRIPTION OF THE DRAWINGS

Figure 1 shows a cross-sectional view of the preferred double plano convex lens.

Figure 2 shows the double plano convex lens from the plano side.

Figure 3 depicts the double plano lens side view with implantation loops.

Figure 4 depicts a cross-section of the human eye with the lens of this invention in place.

Figure 5 shows a cross-section of an implanted lens wherein the patient's posterior lens capsule has been removed in the central area.

## DESCRIPTION OF THE PREFERRED EMBODIMENT

The following detailed description is of the best contemplated mode of carrying out this invention. This description is not to be taken in a limiting sense, but is made merely for the purpose of illustrating the general principles of the invention since the scope of the invention is best defined by the claims appended hereto.

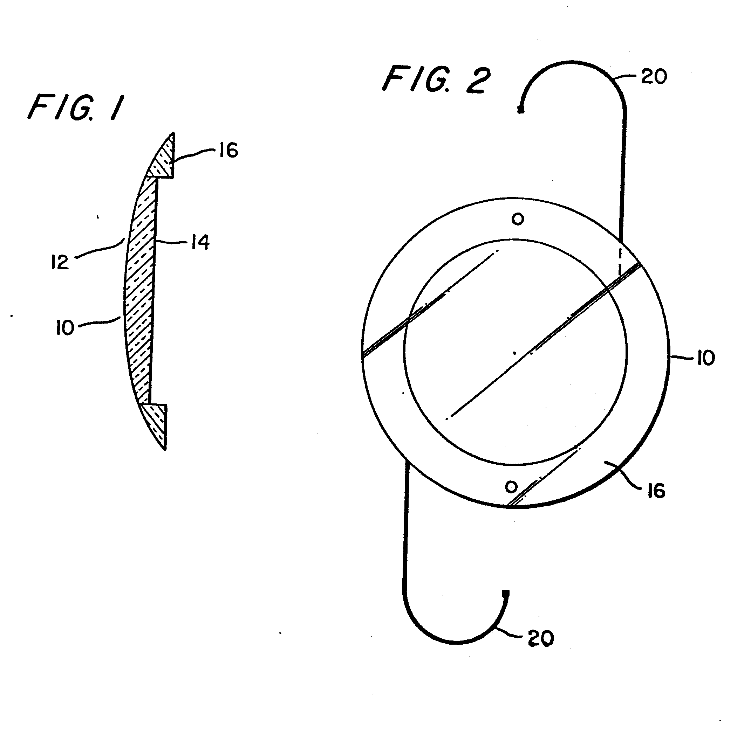

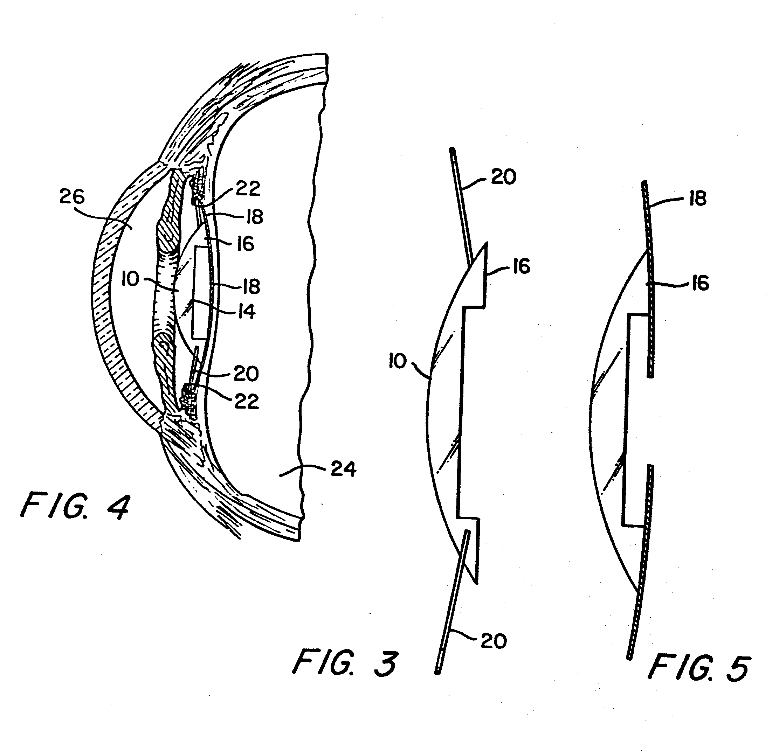

In the preferred embodiment depicted in Figs. 1-5 there is shown a double plano convex lens 10. The lens includes a convex surface 12 a first plano surface 14 which is used for transmission of light to the optic nerve and a second plano surface 16 which provides a means for providing a completely sealed contact between the lens 10 and the patient's posterior lens capsule 18. The second plano surface 16 lies in a plane which is posterior from the first plant surface. The surface 16 extends completely around the periphery of the lens 10 and has no openings. The surface 16 extends generally radially in order to provide the seal with the posterior capsule. The surface 16 is intended to provide a complete seal with the posterior portion of the patient's lens capsule and also to maintain a separation between the lens capsule 16 and the ocular plano surface 14. As can be seen in Figs. 4 and 5, the patient's posterior lens capsule 18 is held away from the lens plano surface 14. The lens plano surface 14 serves as the ocular portion of the lens through which light passes before reaching the retina. Surface 14 is the central portion of the lens.

The lens is also provided with a means for maintaining the lens within the eye. Any suitable known retaining means may be used, such as a Shearing type (J loop), a Sinsky, a Sinsky modified J loop, and a Simcoe type C loop. The loops 20 may also be provided with different degrees of angulation.

The material for the lens 10 and the loop 20 may be polymethylmethacrylate (PMMA) and it may be fabricated by a lathe cut followed by hand polishing. Typically this loop is inclined forward 10° as shwon in Figure 3. A typical lens 10 may have an anterior convex surface 12 which is 6mm, a posterior surface, or a flat plano surface 14, with a 4.5mm optic surface 14 and an outside rim, or second plano surface 16, which is .75mm across to provide for complete contact with the patient's posterior capsule 18. A 4.0mm optic with a second plane surface 1.0mm across may also be used.

An intraocular lens implantation in accordance with this invention requires the steps of removing the anterior portion of the patient's lens capsule 22 and leaving the patient's posterior lens capsule 18 intact. The next step is the insertion of the intraocular lens having a posterior surface 16 which rearward of the optical plano surface 14 for maintaining completely sealed contact with the patient's posterior lens capsule. If in the course of this procedure or later on it is determined that the patient's posterior lens capsule 18 is damaged or has become clouded, than a further step of removing a portion of a patient's posterior lens by directing a laser beam at the lens capsule may be performed. A typical laser beam for such an application is known as a YAG laser. Discission may also be perfomred by surgical discission by what is known as the pars plana technique. The laser discission is preformed after the lens implantation is completed.

In the preferred embodiment it has also been found that a space between the intraocular lens 10 and the patient's posterior capsule 18 can be filled with a balanced saline solution during the implant procedure. Since the posterior capsule 18 is a permeable membrane, the saline solution will subsequently be replaced by the normal ocular fluid.

In this invention a complete sealing contact around the periphery of the patient's intraocular lens is maintained at all times, even after discission is performed by the use of a laser after the initial intraocular transplant. The purpose of maintaining this continuous seal is to prevent movement of the vitreous fluid in the region 24 forward in the patient's eye to the region of the iris 26.

An advantage achieved by this invention is the avoidance of any procedure wherein the posterior portion of the patient's eye is disturbed during lens implantation, or during discission of the patient's posterior lens capsule.

WHAT IS CLAIMED IS:

1.    An intraocular lens implant comprising in combination:

an intraocular lens for transmitting light to the optic nerve of the user having an anterior and posterior surfaces;

means for securing said lens within the eye of the patient;

means for maintaining a completely sealed contact around the periphery of said intraocular lens with the patient's posterior capsule, positioned on the posterior portion of said lens; and

means for maintaining separation between the patient's posterior capsule and the central region of said optical lens where light is transmitted to the patient's retina.

2.    The apparatus of claim 1 wherein said means for maintaining completely sealed contact comprises a surface which is located posterially from said posterior portion of intraocular lens.

3.    The intraocular lens of claim 1 wherein said anterior surface of said intraocular lens is a convex surface.

4.    The intraocular lens of claim 1 wherein said posterior surface is plano.

5.    The intraocular lens of claim 1 wherein said means for maintaining sealed contact with the user's posterior capsule provides a surface which extends generally radially and which is located on the outer circumferential portion of said posterior of said optical lens.

6.    An intraocular lens in accordance with claim 1 wherein said anterior surface is convex, said posterior surface is plano, and said means for maintaining contact with the patient's posterior capsule is plano.

7. An intraocular lens in accordance with claim 1 wherein said posterior surface is a double plano lens surface having first and second plano surfaces.

8. An intraocular lens in accordance with claim 7 wherein said second posterior plano surface is in a plane which is posterior from said first plano surface and which it lies on the outer circumferal edge of said lens.

9. A method of intraocular lens implantation comprising the steps of:

removing the anterior portion of the patient's lens capsule and leaving the posterior portion of the patient's lens capsule intact, and

inserting an intraocular lens having a posterior surface for maintaining a continuous completely sealed contact around the periphery of said intraocular lens with said patient's posterior lens capsule and for maintaining a separation between the patient's posterior lens capsule and the ocular portion of the implanted lens.

10. Method in accordance with claim 9 further including the step of placing and maintaining a fluid between said intraocular lens and that portion of the patient's posterior lens which is not in contact with said intraocular lens.

11. The method of claim 10 further including the step of removing a portion of said patient's posterior lens after completion of the implant procedure by directing a laser beam at said patient's posterior lens capsule.

12. The method in accordance with claim 9 wherein said intraocular lens is a double plano-convex lens, said plano surfaces are on posterior portion of said intraocular lens, and the patient's posterior lens portion maintains a sealing contact with the plano surface which is at the periphery of said intraocular lens.

13. The method in accordance with claim 11 wherein the remaining portion of said patient's posterior lens remains in complete sealing contact around the periphery of said intraocular lens after said portion has been removed by directing said laser beam at said patient's capsule.

14. The method of claim 10 further including the step of performing a surgical discission by the pars plana technique.

FIG. 1

FIG. 2

0134489

FIG. 4

FIG. 3

FIG. 5

0134489

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | US-A-4 244 060 (HOFFER) <br> * Column 8, lines 52-61; figure 5 * | 1-5 | A 61 F  2/16 |
| P,X | WO-A-8 303 753 (MYERS) <br> * Claim 1; figure 5 * | 1 | |
| P,X | WO-A-8 400 883 (CASTLEMAN) <br> * Claim 1; figures 10, 11 * | 1 | |
| D,A | US-A-4 110 848 (JENSEN) | | |
| D,A | US-A-4 328 595 (SHEETS) | | |
| D,A | US-A-4 340 979 (KELMAN) | | **TECHNICAL FIELDS SEARCHED (Int. Cl. ³)** <br><br> A 61 F  2/00 |

The present search report has been drawn up for all claims

| Place of search | Date of completion of the search | Examiner |
|---|---|---|
| BERLIN | 05-10-1984 | KANAL P K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82